# FASCICULE DE BREVET EUROPEEN

(11) **EP 1 263 846 B1**
(45) Date de publication et mention de la délivrance du brevet: **14.04.2004**
(21) Numéro de dépôt: 01907814.6
(22) Date de dépôt: 15.02.2001
(51) Int. Cl.: C08J 3/12, C08J 3/14, C08J 3/16

(54) **BIOPOLYMERE DISPERSABLE ET A HYDRATATION RAPIDE**
DISPERGIERBARES UND SCHNELL HYDRATISIERBARES BIOPOLYMER
DISPERSIBLE AND FAST HYDRATING BIOPOLYMER

(30) Priorité: 18.02.2000 FR 0002037
(43) Date de publication de la demande: 11.12.2002
(73) Titulaire: RHODIA CHIMIE, 92512 Boulogne Billancourt Cedex (FR)
(72) Inventeur: VASLIN, Sophie, F-92210 Saint-Cloud (FR); LYOTHIER, Arnaud, F-93300 Aubervilliers (FR)
(74) Mandataire: Fabre, Madeleine-France
(86) Numéro de dépôt international: PCT/FR2001/000454
(87) Numéro de publication internationale: WO 2001/062831

(56) Documents cités:
- EP-A- 0 180 366
- EP-A- 0 254 603
- EP-A- 0 658 596
- GB-A- 1 547 030

## Description

La présente invention a pour objet des biopolymères, sous forme sèche, dispersables et à hydratation rapide, et leur utilisation comme agents épaississants, émulsifiants et/ou stabilisants pour la préparation de formulations industrielles (comme par exemple les industries du bâtiment, de la peinture, du papier, du textile, des phytosanitaires, du traitement des eaux, industrie pétrolière), alimentaires, cosmétiques, agrochimique et pharmaceutiques.

Les biopolymères de poids moléculaire élevé, qui sont, dans le cadre de la présente invention des polysaccharides, sont utilisés de manière croissante dans de nombreuses applications industrielles pour leurs propriétés épaississantes ou viscosifiantes, émulsifiantes et/ou stabilisantes dans les milieux notamment aqueux. Ainsi, la gomme xanthane, en raison de ses propriétés rhéologiques exceptionnelles, est utilisée dans des domaines aussi variés que le bâtiment, la peinture, le papier, le textile, les cosmétiques, l'alimentaire, l'agriculture, le traitement des eaux, le forage, la récupération du pétrole.

Pour de nombreuses applications, lorsque le biopolymère est sous forme sèche, il est nécessaire de le mettre en solution aqueuse. Or, l'inconvénient majeur de ces poudres est leur difficulté à se disperser et à s'hydrater rapidement dans un milieu aqueux donné. Souvent la dispersion et/ou l'hydratation de ces biopolymères entraîne la formation des grumeaux, lesquels sont préjudiciables à la fonctionnalité du milieu.

Dans le cas d'un biopolymère épaississant comme le xanthane, l'amélioration en termes de dispersion et d'hydratation sans la formation de grumeaux permet notamment une meilleure maîtrise de la viscosité du milieu réactionnel.

C'est la raison pour laquelle il serait avantageux de développer un biopolymère, sous forme sèche, notamment de type gomme xanthane, pouvant être facilement dispersé et rapidement hydraté en milieux aqueux tout en diminuant voire éliminant la formation des grumeaux.

Dans la pratique, l'une des solutions préconisées pour éviter la formation des grumeaux tout en maintenant une bonne dispersabilité et hydratabilité des poudres, consiste à modifier la poudre de polymère par la méthode dite de « granulation ». Cette méthode consiste à agglomérer la poudre de biopolymère en milieu légèrement humide (technique de "lit fluide") afin de fournir des granulés dont la taille moyenne est comprise entre 300 et 1000 microns (voir FR-A-2 600 267). L'inconvénient majeur de cette méthode réside dans son coût élevé de mise en oeuvre.

Une autre solution consiste à additiver le biopolymère avec un composé de type tensioactif (voir EP-A-0 658 596). Toutefois, l'ajout de tels composés ne correspond pas à la tendance actuelle qui est de réduire le nombre d'additifs ajoutés dans une formulation donnée, en particulier dans le domaine alimentaire.

Le but de la présente invention est de proposer une poudre de biopolymère, qui, en milieux aqueux, sans avoir recours à l'ajout d'additif et/ou à de moyens agitation forts, présente une dispersion améliorée et une hydratation rapide tout en diminuant voire éliminant la formation de grumeaux.

A cet effet, l'invention a pour objet un biopolymère, sous forme sèche, dispersable et à hydratation rapide dont au moins 75 % en poids des particules présentent un diamètre compris entre 60 et 250 microns et un diamètre moyen (d₅₀) compris entre 100 et 250 microns.

De préférence, au moins 75 % en poids des particules du biopolymère présentent un diamètre compris entre 100 et 200 microns et un diamètre moyen (d₅₀) compris entre 100 et 200 microns.

Le choix astucieux de la distribution granulométrique des biopolymères selon l'invention permet à la fois de mieux contrôler la dispersion de la poudre et de favoriser son hydratation tout en réduisant sensiblement et le plus souvent en supprimant totalement la formation des grumeaux.

Les biopolymères de l'invention présentent, en outre, l'avantage d'être sans poussière et facilement coulable.

D'autres avantages et caractéristiques de la présente invention apparaîtront clairement à la lecture de la description, des exemples et des figures qui vont suivre.

Dans la présente description, la notation d₅₀ représente la répartition de la taille des particules telle que 50 % en volume des particules ont une taille inférieure ou égale à ladite taille. Par exemple, un d₅₀ de 100 microns signifie que 50 % en volume des particules ont une taille inférieure ou égale à 100 microns.

La granulométrie est déterminée par spectroscopie laser avec un appareil de type « Coulter LS 230 » configuration voie sèche.

Dans le cadre de la présente invention, le terme « biopolymère » désigne plus particulièrement des polysaccharides de poids moléculaires élevés, le plus souvent supérieurs à 1.10⁶ g/mol (mesurés par perméation de gel), et qui sont constitués d'unités de glucose, mannose, galactose, rhamnose, acide glucuronique, acide mannuronique, acide guluronique, avec éventuellement des dérivés acétate et pyruvate. Leur structure particulière et leurs propriétés sont décrites par exemple dans l'ouvrage Industrial Gums - Whistler - 2^{nd} Edition - Chapters XXI-XXIII (1973).

Ces biopolymères sont produits avantageusement par culture aérobie de microorganismes dans un milieu nutritif aqueux.

De nombreux microorganismes tels que bactéries, levures, champignons, algues, sont capables de produire ces biopolymères. On peut citer entre autres :
■ des bactéries appartenant au genre *Xanthomonas* et plus particulièrement aux espèces décrites dans Bergey's Manual of Determinative Bacteriology (8^{e} édition - 1974 - Williams N. Wilkins Co. Baltimore) telles que *Xanthomonas begoniae, Xanthomonas campestris, Xanthomonas carotae, Xanthomonas hederae, Xanthomonas incanae, Xanthomonas malvacearum, Xanthomonas papavericola, Xanthomonas phaseoli, Xanthomonas pisi, Xanthomonas vasculorum, Xanthomonas vesicatoria, Xanthomonas vitians, Xanthomonas pelargonii* ;
■ des bactéries appartenant au genre *Arthrobacter* et plus particulièrement les espèces *Arthrobacter stabilis, Arthrobacter viscosus* ;
■ des bactéries appartenant au genre *Erwinina* ;
■ des bactéries appartenant au genre *Azotobacter* et plus particulièrement l'espèce *Azotobacter indicus* ;
■ des bactéries appartenant au genre *Agrobacterium* et plus particulièrement les espèces *Agrobacterium radiobacter, Agrobacterium rhizogenes, Agrobacterium tumefaciens ;*
■ des bactéries appartenant au genre *Alcaligenes* et plus particulièrement *Alcaligenes faecalis* ;
■ des bactéries appartenant au genre *Pseudomonas* et plus particulièrement *Pseudomonas methanica* ;
■ des bactéries appartenant au genre *Corynebacterium* ;
■ des bactéries appartenant au genre *Bacillus* et plus particulièrement *Bacillus polymyxa* ;
■ des champignons appartenant au genre *Sclerotium* et plus particulièrement aux espèces *Sclerotium glucanicum, Sclerotium rolfsii* ou *Plectania occidentalis* ;
■ des champignons appartenant au genre *Aspergillus* et plus particulièrement aux espèces *Aspergillus itaconicus, Aspergillus terreus* ;
■ des levures appartenant au genre *Hansenula* comme l'espèce *Hansenula capsulata*.

Dans un mode préféré de l'invention, le microorganisme est une bactérie du genre *Xanthomonas*, plus particulièrement de l'espèce *Xanthomonas campestris*, et le biopolymère est la gomme xanthane.

Les biopolymères selon l'invention peuvent être obtenus par tout procédé permettant de maîtriser la répartition granulométrique d'une poudre. A titre d'exemple, on peut citer le tamisage.

Comme déjà indiqué, les biopolymères selon la présente invention se dispersent facilement en milieux aqueux et ne requièrent pas de moyens de cisaillement importants. La dispersabilité de la poudre de biopolymère s'évalue par le comptage des grumeaux formés lorsque le biopolymère est mis en solution aqueux. Plus il y a de grumeaux formés, moins bonne sera la dispersabilité.

La vitesse d'hydratation peut être déterminée par exemple en mesurant la viscosité développée au cours du temps dans des conditions d'agitation douce. A titre indicatif, une agitation douce peut correspondre à une agitation avec une pale défloculeuse avec une vitesse pouvant être comprise entre 400 et 600 tr/min.

Ces caractéristiques permettent d'incorporer les biopolymères selon l'invention dans de nombreuses formulations, comme agent épaississant ou viscosant, émulsifiant, et/ou stabilisant.

L'invention couvre, en outre, l'utilisation d'un biopolymère tel que défini précédemment comme agent épaississant ou viscosant, émulsifiant, et/ou stabilisant dans les formulations :
- pour le forage, la récupération assistée du pétrole, le traitement des eaux ;
- pour le papier, le bâtiment, le textile ;
- alimentaires, cosmétiques, agrochimiques, pharmaceutiques ;
- pour nettoyage industriel et ménager ;
ainsi que lesdites formulations contenant de tel biopolymère.

Les exemples présentés ci-après sont à titre illustratif et non limitatif de la présente invention.

### Légende des figures :

La figure 1 représente la répartition granulométrique des échantillons : produits A, B et C, obtenu à l'aide d'un granulomètre Coulter LS230 en configuration voie sèche.
La figure 2 représente la vitesse d'hydratation des échantillons A, B et C, dans l'eau distillée. Cette vitesse est déterminée par la mesure de la viscosité développée au cours du temps dans des conditions de l'exemple 1 - Test d'hydratation dans l'eau distillée.
La figure 3 représente la vitesse d'hydratation des échantillons A, B et C, dans un milieu à 40 % de saccharose. Cette vitesse est déterminée par la mesure de la viscosité développée au cours du temps dans des conditions de l'exemple 2.

### EXEMPLES

### Exemple 1

### ■ Préparation des échantillons

Produit A : Le produit A est une poudre de gomme xanthane commerciale standard vendue sous le nom de Rhodigel© par la société Rhodia.
Produit B : Le produit B est selon l'invention.
   Il s'agit d'une gomme xanthane dont la taille des particules est comprise entre 180 µm et supérieure à 125 µm. Ce produit est obtenu par deux tamisages simultanés : un tamisage à travers un tamis de 180 µm d'ouverture de maille et un tamisage à travers un tamis de 125 µm.
Produit C : Le produit C est une poudre de gomme xanthane, ayant subi une granulation par la technique dite « de lit fluide » selon l'exemple 1 du brevet FR 2 600 267.

**Tableau 1**

| **Echantillon** | **Produit A** | **Produit B** | **Produit C** |
|---|---|---|---|
| % de particules comprises entre 60 et 250 microns | 68 | 98 | 38 |
| % de particules comprises entre 100 et 200 microns | 48 | 79 | 18 |
| d ₅₀ microns | 149,6 | 180,8 | 177,1 |

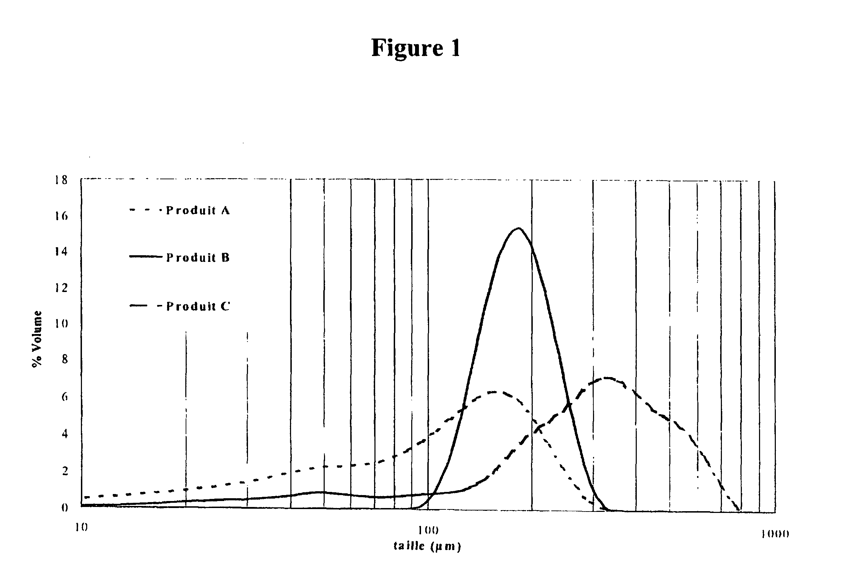

La figure 1 représente la répartition granulométrique des échantillons A, B et C.

### ■ Test de dispersabilité

Les propriétés de dispersion d'un hydrocolloïde peuvent être évaluées par un test permettant le décompte du nombre de grumeaux présents dans une solution après dispersion.

On prépare des solutions de gomme xanthane à 0,3% dans l'eau distillée. Cette mise en solution est réalisé sur une quantité de 500 ml d'eau distillée, dans un bécher de 1000 ml de forme basse, avec une pale défloculeuse de 65 mm de diamètre, avec une vitesse de 400 tr/min pendant 15 min.

Ladite solution est filtrée sur un tamis de 1 mm d'ouverture de maille et le nombre de grumeaux présents sur le tamis après filtration est relevé.

Les résultats sont récapitulés dans le tableau 2.

**Tableau 2**

| **Produit** | **Nombre de grumeaux** |
|---|---|
| Produit A | 55 |
| Produit B | 0 |
| Produit C | 0 |

Un produit est considéré comme
- « TRES BON » si le nombre de grumeaux est inférieur à 5 ;
- « BON » si le nombre de grumeaux est inférieur à 10 ;
- MAUVAIS » di le nombre de grumeaux est supérieur à 10.

### ■ Test d'hydratation

Ce test permet d'évaluer la vitesse d'hydratation d'une poudre de biopolymère en milieux aqueux.

On met en solution 0,3 % de poudre de gomme de xanthane (produits A, B, C) dans l'eau distillée. Cette mise en solution est réalisée sur une quantité de 500 ml d'eau distillée, dans un bécher de 1000 ml de forme basse, avec une pale défloculeuse de 65 mm de diamètre, avec une vitesse de 400 tr/min pendant 15 min. On mesure la viscosité de ladite solution à quatre moments différents, à l'aide d'un viscosimètre Brookfield modèle LVT, broche n°2, réglé à 12 tr/min. Ces quatre moments sont à 1, 2, 5 et 15 minutes.

Les valeurs de viscosité figurent dans le tableau 3 suivant :

**Tableau 3**

| **Produit** | **Viscosité à 1 min** | **Viscosité à 2 min** | **Viscosité à 5 min** | **Viscosité à 15 min** |
|---|---|---|---|---|
| Produit A | 20 mPa.s | 100 mPa.s | 332 mPa.s | 605 mPa.s |
| Produit B | 238 mPa.s | 692 mPa.s | 747 mPa.s | 742 mPa.s |
| Produit C | 137 mPa.s | 712 mPa.s | 687 mPa.s | 675 mPa.s |

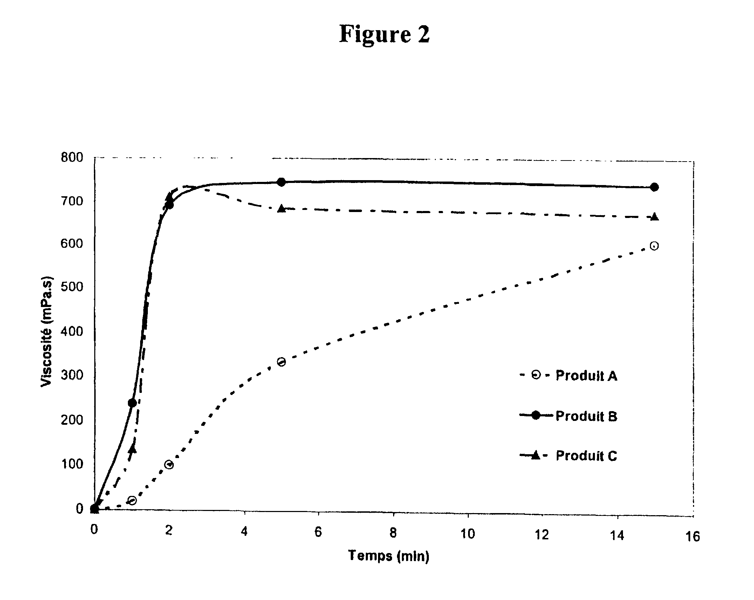

La figure 2 représente la vitesse d'hydratation des échantillons A, B et C, dans l'eau distillée. Cette vitesse est déterminée par la mesure de la viscosité développée au cours du temps.

### Exemple 2

### Test d'hydratation dans une solution à 40% saccharose

Ce test permet d'évaluer la vitesse d'hydratation d'une poudre de biopolymère en milieux aqueux sucré.

On met en solution 0,3% de poudre de gomme de xanthane (produits A, B, C) dans une solution contenant 40 % de saccharose. Cette mise en solution est réalisée sur une quantité de 500 g de solution à 40% de sucre, dans un bécher de 1000 ml de forme basse, avec une pale défloculeuse de 65 mm de diamètre, avec une vitesse de 400 tours/minutes pendant 30 minutes. On mesure la viscosité de ladite solution à trois moments différents, à l'aide d'un viscosimètre Brookfield modèle LVT, broche n°2, réglé à 12 tr/min. Ces trois moments sont à 5, 15 et 30 minutes.

Les valeurs de viscosité figurent dans le tableau 4.

**Tableau 4**

| **Produit** | **Viscosité à 5 min** | **Viscosité à 15min** | **Viscosité à 30min** |
|---|---|---|---|
| Produit A | 870 mPa.s | 1140 mPa.s | 1280 mPa.s |
| Produit B | 970 mPa.s | 1470 mPa.s | 1480 mPa.s |
| Produit C | 1075 mPa.s | 1430 mPa.s | 1410 mPa.s |

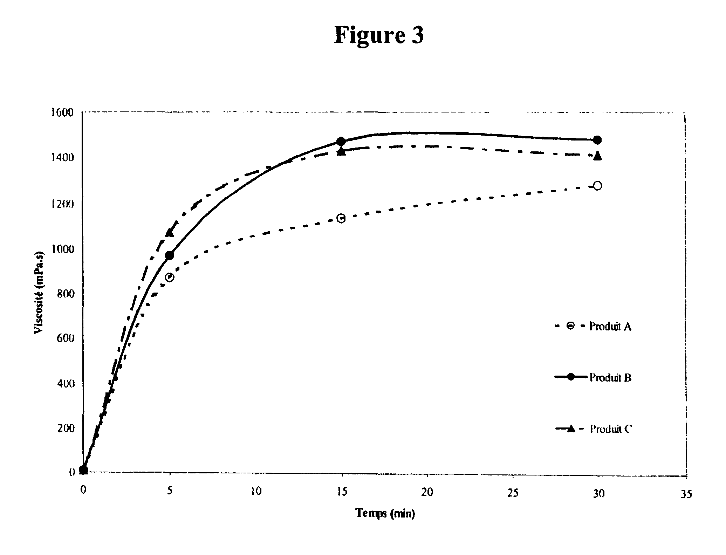

La figure 3 représente la vitesse d'hydratation des échantillons A, B et C, dans un milieu à 40 % de saccharose. Cette vitesse est déterminée par la mesure de la viscosité développée au cours du temps.

## Revendications

1. Biopolymère sous forme sèche, dispersable et à hydratation rapide **caractérisé en ce qu'**au moins 75 % en poids des particules dudit biopolymère présentent un diamètre compris entre 60 et 250 microns et un diamètre moyen (d₅₀) compris entre 100 et 250 microns, la notation d₅₀ représentant la répartition de la taille des particules telle que 50 % en volume des particules ont une taille inférieure ou égale à ladite taille.

2. Biopolymère selon la revendication 1, **caractérisé en ce qu'**au moins 75 % en poids des particules dudit biopolymère présentent un diamètre compris entre 100 et 200 microns et un diamètre moyen (d₅₀) compris entre 100 et 200 microns.

3. Biopolymère selon l'une des revendications 1 ou 2, **caractérisé en ce que** ledit biopolymère est une gomme xanthane.

4. Utilisation d'un biopolymère selon l'une quelconque des revendications 1 à 3 comme agent épaississant ou viscosant, émulsifiant, et/ou stabilisant.

5. Utilisation d'un biopolymère selon l'une quelconque des revendications 1 à 3 comme agent épaississant ou viscosant, émulsifiant, et/ou stabilisant dans les formulations pour le forage, la récupération assistée du pétrole, le traitement des eaux.

6. Utilisation d'un biopolymère selon l'une quelconque des revendications 1 à 3 comme agent épaississant ou viscosant, émulsifiant, et/ou stabilisant dans des formulations alimentaires, cosmétiques, pharmaceutiques, agrochimiques.

7. Utilisation d'un biopolymère selon l'une quelconque des revendications 1 à 3 comme agent épaississant ou viscosant, émulsifiant, et/ou stabilisant dans des formulations pour nettoyage industriel et ménager.

8. Utilisation d'un biopolymère selon l'une quelconque des revendications 1 à 3 comme agent épaississant ou viscosant, émulsifiant, et/ou stabilisant dans des formulations pour le papier, le bâtiment, le textile.

9. Utilisation d'un biopolymère selon l'une quelconque des revendications 1 à 3 pour réaliser une dispersion en milieux aqueux sans avoir recours à l'ajout d'additif.

10. Formulation pour le forage, la récupération assistée du pétrole, le traitement des eaux, alimentaire, cosmétique, pharmaceutique, agrochimique, pour le nettoyage industriel et ménager, pour le papier, le bâtiment, le textile, utilisant un biopolymère selon l'une quelconque des revendications 1 à 3, comme agent épaississant ou viscosant, émulsifiant, et/ou stabilisant.

## Patentansprüche

1. Biopolymer in Trockenform, dispergierbar und mit rascher Hydratation, **dadurch gekennzeichnet, dass** wenigstens 75 Gew.-% der Teilchen des Biopolymers einen Durchmesser, der zwischen 60 und 250 Mikrometer liegt, und einen mittleren Durchmesser (d₅₀), der zwischen 100 und 250 Mikrometer liegt, aufweisen, wobei der Begriff d₅₀ die Größenverteilung der Teilchen derart darstellt, dass 50 Volumen-% der Teilchen eine Größe kleiner oder gleich dieser Größe aufweisen.

2. Biopolymer gemäß Anspruch 1, **dadurch gekennzeichnet, dass** wenigstens 75 Gew.-% der Teilchen des Biopolymers einen Durchmesser, der zwischen 100 und 200 Mikrometer liegt, und einen mittleren Durchmesser (d₅₀), der zwischen 100 und 200 Mikrometer liegt, aufweisen.

3. Biopolymer gemäß Anspruch 1 oder 2, **dadurch gekennzeichnet, dass** das Biopolymer ein Xanthangummi ist.

4. Verwendung eines Biopolymers gemäß einem der Ansprüche 1 bis 3 als Verdickungsmittel oder Eindickmittel, als Emulgierungsmittel und/oder als Stabilisierungsmittel.

5. Verwendung eines Biopolymers gemäß einem der Ansprüche 1 bis 3 als Verdickungsmittel oder Eindickmittel, als Emulgierungsmittel und/oder als Stabilisierungsmittel in Formulierungen für Bohrung, Erdölgewinnung, Wasseraufbereitung.

6. Verwendung eines Biopolymeren gemäß einem der Ansprüche 1 bis 3 als Verdickungsmittel oder Eindickmittel, als Emulgierungsmittel und/oder als Stabilisierungsmittel in Nahrungsmittelformulierungen, kosmetischen, pharmazeutischen, agrochemischen Formulierungen.

7. Verwendung eines Biopolymeren gemäß einem der Ansprüche 1 bis 3 als Verdickungsmittel oder Eindickmittel, als Emulgierungsmittel und/oder als Stabilisierungsmittel in Formulierungen für Industrie- oder Haushaltsreinigung.

8. Verwendung eines Biopolymeren gemäß einem der Ansprüche 1 bis 3 als Verdickungsmittel oder Endickmittel, als Emulgierungsmittel und/oder als Stabilisierungsmittel in Formulierungen für Papier, Bauten, Textilien.

9. Verwendung eines Biopolymeren gemäß einem der Ansprüche 1 bis 3 zur Erstellung einer Dispersion in flussiges media ohne auf die Zugabe von Additiv zurückzugreifen.

10. Formulierung für Bohrung, Erdölgewinnung, Wasseraufbereitung, Nahrungsmittelformulierung, kosmetische Formulierung, pharmazeutische Formulierung, agrochemische Formulierung, für Industrie- oder Haushaltsreinigung, für Papier, Bauten, Textilien, wobei ein Biopolymer gemäß einem der Ansprüche 1 bis 3 verwendet wird, als Verdickungsmittel oder Eindickmittel, als Emulgierungsmittel und/oder als Stabilisierungsmittel.

## Claims

1. Dispersible and fast-hydrating biopolymer in dry form, **characterized in that** at least 75% by weight of the particles of the said biopolymer have a diameter of between 60 and 250 microns and a mean diameter (d₅₀) of between 100 and 250 microns, the notation d₅₀ represents the particle size distribution such that 50% by volume of the particles are less than or equal to the said size

2. Biopolymer according to claim 1, **characterized in that** at least 75% by weight of the particles of the said biopolymer have a diameter of between 100 and 200 microns and a mean diameter (d₅₀) of between 100 and 200 microns.

3. Biopolymer according to either of claims 1 and 2, **characterized in that** the said biopolymer is a xanthan gum.

4. Use of a biopolymer according to any one of claims 1 to 3 as a thickener, viscosity modifier, emulsifier and/or stabilizer.

5. Use of a biopolymer according to any one of claims 1 to 3 as a thickener, viscosity modifier, emulsifier and/or stabilizer in formulations for the drilling for and the assisted recovery of petroleum and for water treatment.

6. Use of a biopolymer according to any one of claims 1 to 3 as a thickener, viscosity modifier, emulsifier and/or stabilizer in food, cosmetics, pharmaceutical and agrochemical formulations.

7. Use of a biopolymer according to any one of claims 1 to 3 as a thickener, viscosity modifier, emulsifier and/or stabilizer in formulations for industrial and household cleaning.

8. Use of a biopolymer according to any one of claims 1 to 3 as a thickener, viscosity modifier, emulsifier and/or stabilizer in formulations for paper, construction and textiles.

9. Use of a biopolymer according to any one of claims 1 to 3 to realise a dispersion in aqueous media without the need to add an additive.

10. Formulation for drilling for and assisted recovery of petroleum, for water treatment, food, cosmetics, pharmaceuticals or agrochemicals, for industrial and household cleaning or for paper, construction or textiles, using a biopolymer according to any one of claims 1 to 3, as a thickener, viscosity modifier, emulsifier and/or stabilizer.
